# EUROPEAN PATENT APPLICATION

(11) **EP 3 482 688 A1**
(43) Date of publication of application: **15.05.2019**
(21) Application number: 17200616.5
(22) Date of filing: 08.11.2017
(51) Int. Cl.: A61B 6/00

(54) **DEVICE AND METHOD FOR DETERMINING IMAGE PARAMETERS FOR GENERATING AN X-RAY PULSE**

(71) Applicant: Koninklijke Philips N.V., 5656 AE Eindhoven (NL)
(72) Inventor: ENGEL, Klaus Juergen, 5656 AE Eindhoven (NL); VAN DAAL, François Cornelius Johannes, 5656 AE Eindhoven (NL); FEDDES, Bastiaan, 5656 AE Eindhoven (NL); MENSER, Bernd, 5656 AE Eindhoven (NL); WISSINK, Gerhardus Johannes, 5656 AE Eindhoven (NL)
(74) Representative: de Haan, Poul Erik

(57) **Abstract**

The present invention relates to a device for determining a focal spot size, , and/or a pulse duration, and/or an X-ray intensity for an X-ray pulse for a sequential X-ray imaging apparatus, the device (1) comprising: a receiving unit (2); a mode determining unit (3); and a transmitting unit (4); wherein the receiving unit (2) is configured to receive a status signal (24) indicating a motion status of an object of interest (7); wherein the mode determining unit (3) is configured to determine an acquisition mode based on the indicated motion status of the object of interest (7); wherein the acquisition mode defines a focal spot size, and/or a pulse duration, and/or an X-ray intensity for an X-ray pulse for a sequential X-ray imaging apparatus (10); wherein the focal spot size, and/or the pulse duration, and/or X-ray intensity for an X-ray pulse are adapted to the motion status of the object of interest (7); and wherein the transmitting unit (4) is configured to provide a mode signal indicating the determined acquisition mode. The invention reduces a focal spot blurring and/or a temporal smearing for the motion phases of a moving object to be imaged with X-ray pulses during a minimal invasive procedure.

## Description

### FIELD OF THE INVENTION

The present invention relates to a device and a method for determining image parameters for generating an X-ray pulse.

### BACKGROUND OF THE INVENTION

In minimal invasive interventional procedures, fluoroscopic X-ray imaging is used to control the interaction of any device introduced into the body, e.g. a catheter carrying a stent in a beating heart.

One of the most important image quality parameters is the spatial resolution of the X-ray image, combined with a reasonable high contrast-to-noise ratio. The image resolution depends on spatial aspects, i.e. depending on the size of the focal spot in the X-ray tube and the detector resolution, but also temporal aspects, i.e. depending on the length of the X-ray exposure per image frame and the velocity of an imaged object, e.g. a stent.

The detector resolution is a fixed parameter, but the focal spot size of the electron beam in the X-ray tube can be varied. Typical commercial tube systems provide a small and a large focal spot, with "small" being in the range of 0.3 mm to 0.5 mm effective size, and "large" in the range of 0.7 mm to 1.2 mm. The small focal spot provides a better spatial image resolution on the disadvantage of a lower available X-ray power than a large focal spot, since the thermal load can be distributed over a larger area in a large focal spot.

If an object is moving, it is smeared out during image acquisition. Therefore, it is mandatory to reduce the exposure time to optimize spatial resolution. Practically, it turns out that e.g. cardiac imaging needs pulse times of not more than a few milliseconds to still deliver acceptable image quality. For this reason, cardiac imaging is performed in a series of fluoroscopic X-ray exposures at a frame rate of for example 30 frames per second, with pulse lengths for individual images in the millisecond range.

To maintain a reasonable well contrast-to-noise ratio, a certain dose needs to be delivered to the detector per X-ray pulse. This is typically done in a feedback loop; the last acquired image is analyzed for a dose level which is compared to a pre-defined reference dose. If the dose level is too low or too high, the X-ray dose for the next pulse may then be adjusted. This can be repeated until finally the reference dose level is reached.

The dose can be regulated by adjusting the three parameters tube voltage, tube current, and pulse time. An example is known from US 8,971,493 B2 which shows an adaptive dose optimization using X-ray beam spatial control and beam exposure time gating and triggering in response to hemodynamic, electrophysiological and vital sign signals. The signals enable adaptive variation in timing of image acquisition.

However, there are some restrictions. The electrical power, i.e. the product of tube voltage and tube current, has an upper limit to protect the anode surface from local thermal damage. This upper limit depends on the usage of a small or a large focal spot and the pulse time. Furthermore, the tube current is a very slowly adaptable quantity.

Typically, the X-ray dose rate control regulates X-ray parameters to a stable triplet of tube voltage/tube current/pulse time.

### SUMMARY OF THE INVENTION

There may thus be a need to provide a device and a method which further improves an X-ray dose rate control. In particular, it may be desirable to minimize focal spot blurring and/or a temporal smearing for motion phases of a moving object to be imaged with X-ray pulses during a minimal invasive procedure.

The object of the present invention is solved by the subject-matter of the independent claims; further embodiments are incorporated in the dependent claims. It should be noted that the following described aspects of the invention apply also for the system and the method.

According to the present invention, a device for determining imaging parameters for generating an X-ray pulse in an X-ray imaging apparatus is provided, the device comprising a receiving unit configured to receive a status signal indicative of a motion status of an object of interest, and a mode determining unit configured to determine an acquisition mode defining one or more imaging parameters for generating the X-ray pulse, wherein the one or more imaging parameters are defined in dependence of the indicated motion status of the object of interest.

In an embodiment, such device provides an adaptive changing of one or more imaging parameters of the acquisition mode during the pulsed X-ray imaging of an object of interest, which may either be moving or at rest. Preferably, dose settings can be tuned to a rest phase of a moving object, or for the phase of maximum object velocity. The mode determining unit analyzes the motion status of the object of interest being provided by the status signal which has been received by the receiving unit.

Depending on the information provided by the status signal, the mode determining unit determines for an X-ray pulse at least one imaging parameter. For example, one or more imaging parameters selected from the group of: focal spot size, pulse duration and X-ray intensity may be determined. Thus, at least one of the focal spot size, the pulse duration, and the X-ray intensity may be adapted to the motional conditions of the object of interest for each X-ray pulse for the purpose of maximal spatial resolution of the object in each image frame.

Thus, a pulsed acquisition mode for an X-ray imaging apparatus may be adapted to a motion status of an object of interest to be imaged. This means, for each motion phase of an object of interest, an X-ray imaging parameter (or "pulse parameter"), which may for example be at least one of the focal spot size, the pulse length, and the X-ray intensity of an X-ray pulse are chosen such that the focal spot blurring and/or a temporal smearing in an X-ray image of the object of interest to be imaged is minimized.

According to an example, the mode determining unit is configured to provide a smaller focal spot size and a longer pulse duration if the status signal indicates that the object of interest is in a rest phase than if the status signal indicates that the object of interest is in a movement phase.

A small focal spot size and a relatively long pulse duration for a pulsed X-ray image during a rest phase of a moving object of interest may provide a better spatial resolution than a large focal spot size and a relatively short pulse duration. Furthermore, a large focal spot size in combination with a short pulse duration may provide an optimal spatial resolution if it reduces a relevant temporal smearing in the image of a moving object of interest during a movement phase.

According to an example, the mode determining unit is configured to provide a constant dose for all X-ray pulses for all motion statuses. In this respect, "constant dose" shall be construed as including examples wherein minor dose variations, for instance up to 5%, may occur between subsequent pulses.

The X-ray dose may then be kept constant for each pulsed X-ray image of an imaging sequence. This means, that if the tube voltage and the tube current of an X-ray tube, which both may determine the X-ray intensity, are varied, the pulse duration may be varied in opposite direction. This means an increase of the X-ray intensity results in a reduction of the pulse duration. Furthermore, a reduction of the X-ray intensity results in an increase of the pulse duration.

According to an example, the dose of an X-ray pulse is adapted by a pulse duration in combination with an X-ray tube voltage and/or an X-ray tube current.

According to a further aspect, an X-ray imaging system may be provided, comprising an X-ray imaging apparatus; a status acquisition apparatus; and a device according to one of the above described examples and embodiments. In an embodiment, the X-ray imaging apparatus comprises an X-ray tube and a controller. The status acquisition apparatus is configured to determine a motion status of an object of interest and to provide the status signal indicative of the motion status to the device. Furthermore, the controller is configured to control the X-ray tube according to the one or more imaging parameters of the acquisition mode as determined by the device.

In an embodiment, such device provides an adaptive changing of one or more imaging parameters of the acquisition mode during the pulsed X-ray imaging of an object of interest, which may either be moving or at rest.

According to an example, the status acquisition apparatus is an electro-cardiograph (ECG) apparatus.

In this case, the moving object of interest may be a heart which is monitored with the electrocardiograph apparatus. The electrocardiograph apparatus provides an easy and an exact monitoring of a heartbeat such that the motion status of the heart may be acquired with a high accuracy. Furthermore, the analyzation of an electrocardiograph signal may provide a simple determination of the motion status of the heart, i.e. it is easy to see when the heart is in the rest phase or in the movement phase.

Alternative embodiments of a status acquisition apparatus, in particular for deriving a phase of the cardiac cycle, are known in the art and could be implemented instead of an ECG.

According to an example, the controller is configured to adjust a tube voltage of the X-ray tube when controlling an X-ray intensity for the X-ray pulse.

According to another example, the controller is configured to adjust the tube current of the X-ray tube when controlling an X-ray intensity for the X-ray pulse.

According to an example, the status signal indicating a motion status of an object of interest comprises a rest phase segment and/or a movement phase segment.

According to another example the sequential X-ray imaging apparatus is a fluoroscopy device.

According to a further aspect, a method for determining imaging parameters for generating an X-ray pulse in an X-ray imaging apparatus is provided. The method comprises the steps of receiving a status signal indicative of a motion status of an object of interest; and determining an acquisition mode including defining one or more imaging parameters of the acquisition mode in dependence of the indicated motion status of the object of interest.

According to an example, prior to the receiving step, the method further comprises the steps of specifying a region of interest, for example a heart region, at an object of interest via a user interface; and adjusting a status acquisition apparatus, for example an ECG apparatus, to the region of interest.

According to an example, the method further comprises the steps of normalizing the signal in a sequence of X-ray images being acquired in a pulsed acquisition with an X-ray imaging apparatus, using different focal spot sizes and different doses for the respective X-ray pulses; and applying a noise reduction algorithm to the sequence of X-ray images. In particular, the different focal spot sizes and X-ray doses may be defined in an acquisition mode that is determined in dependence of an indicated motion status on an object of interest, for example a phase of a human heart cycle.

X-ray images being acquired with different focal spot sizes and different doses may have different averaged intensities and different signal-to-noise ratios. Therefore, those images may not be immediately compared with each other and show a flickering if viewed in an image sequence. These exemplary steps provide images which may be compared by a user and image sequences with a minimized flickering due to the normalization of the averaged intensity and the noise reduction.

According to a further aspect, a computer program is provided that comprises instructions for controlling an apparatus or system according to the description mentioned above, which instructions, when being executed by a processing unit of a computer, cause the computer to perform the method steps according to the description mentioned above.

According to a further aspect, a computer readable medium is provided having stored such program.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 shows a schematic view of the system for determining a focal spot size and a pulse duration for an X-ray pulse for a sequential X-ray imaging apparatus.
Fig. 2 shows a schematic view of the device for determining a focal spot size and a pulse duration for an X-ray pulse for a sequential X-ray imaging apparatus.
Fig. 3 shows a schematic diagram of the pulse duration over the focal spot size.
Fig. 4a-d shows schematic images of a moving object of interest during different motional phases being imaged with different acquisition modes.
Fig. 5 shows a schematic image of a status signal.
Fig. 6 shows a schematic diagram of the method for determining a focal spot size and a pulse duration for an X-ray pulse for a sequential X-ray imaging apparatus.

### DETAILED DESCRIPTION OF EMBODIMENTS

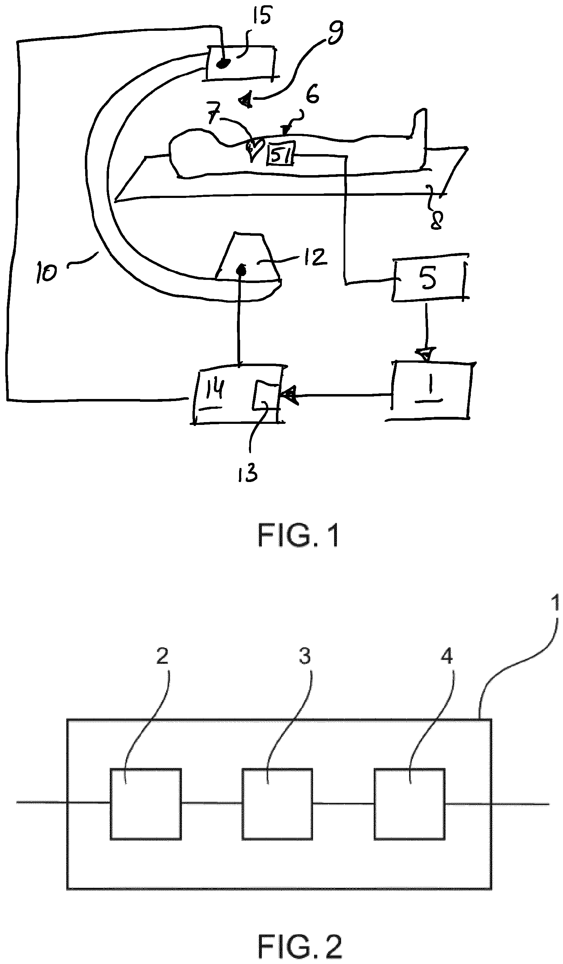

Fig. 1 shows a schematic view of the system 30 for determining imaging parameters, for example a focal spot size and/or a pulse duration and/or an X-ray intensity, for an X-ray pulse for a sequential X-ray imaging apparatus. The system 30 comprises an X-ray imaging apparatus 10 e.g. a C-arm device for acquiring fluoroscopic X-ray images, a status acquisition apparatus 5, and a device 1 for determining the imaging parameters.

In Fig. 1, a patient 6 is arranged on a patient support device 8. In this example, the object of interest 7 is a stent or a catheter being introduced to the patient's cardiac vasculature, which is a moving object. However, also an organ of the patient or any moving non-living object may be an object of interest 7 to be imaged with the present invention.

To image the stent, at least a portion of the chest of the patient 6 is arranged within an object receiving space 9. An object in the object receiving space 9 maybe imaged by the X-ray imaging apparatus 10. The X-ray imaging apparatus 10 may comprise an X-ray tube 12, a controller 14 and an X-ray detector 15. The controller 14 may be provided with a mode signal receiving unit 13. In an exemplary embodiment, the sequential X-ray imaging apparatus 10 maybe a fluoroscopy device.

The X-ray tube 12 emits pulsed X-ray radiation. A focal spot of an electron beam on an anode of the X-ray tube 12 maybe variable in size for the X-ray radiation emitted towards the X-ray detector 15. The variation of the focal spot size may be provided for each X-ray pulse of an imaging sequence. This means, that each X-ray image of a sequentially acquired X-ray image sequence may have a different focal spot size. The X-ray radiation travels through the object being arranged in the object receiving space 9. Then, the X-ray radiation is detected by the X-ray detector 15.

A variation of the focal spot size may, for example, involve a focusing element within the X-ray tube, such as an electron optical lens system that focuses the electron beam traveling between a cathode and a focal spot area on the X-ray tube anode. Thus, a focal spot of a desired size is obtained, from which an X-ray radiation beam is generated. The X-ray radiation then travels through the object of interest 7 to the X-ray detector 15.

A controller 14 controls the acquisition modalities for the X-ray tube 12 and X-ray detector 15. In the previous example, the controller 14 maybe configured to control the focusing element within the X-ray tube.

The status acquisition apparatus 5, which maybe an electrocardiograph apparatus, may comprise a sensor 51 which is connected to the chest of the patient 6 being arranged on a patient support device 8. In the exemplary embodiment being shown in Fig. 1, the status acquisition apparatus 5 monitors the heartbeat of the patient 6. The movement status of the heart may be determined from the monitoring of the heartbeat. The status signal 24 of the electrocardiograph apparatus comprises a movement phase 22, which indicates a movement of the heart during a heartbeat, and a rest phase 23 indicating a non-moving phase of the heart between two heart beats.

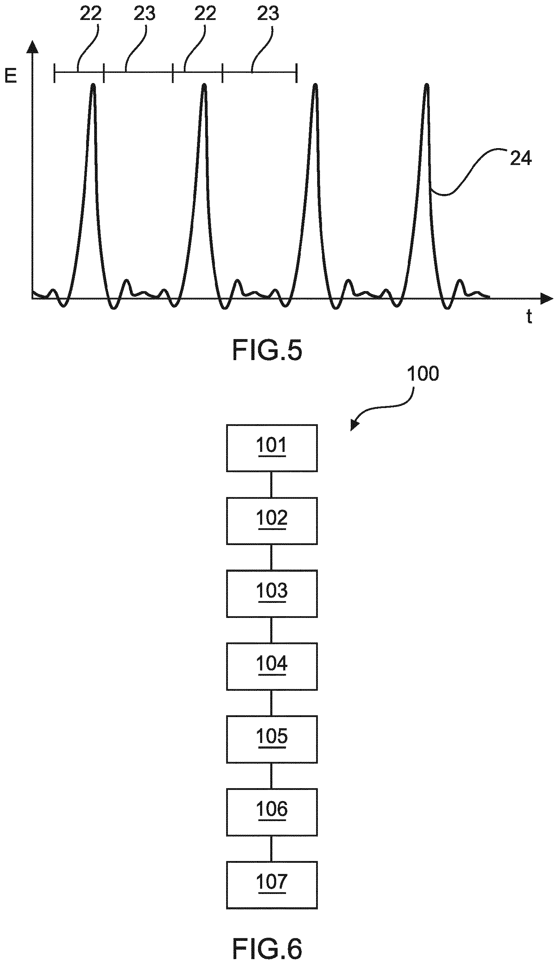

The status acquisition apparatus 5 may provide the signal of the electrocardiograph apparatus as a status signal 24 being shown in Fig. 5. The status signal 24 may be received by the device 1. According to Fig. 2, device 1 comprises a receiving unit 2, a mode determining unit 3, and optionally a transmitting unit 4. The receiving unit 2 may receive the status signal 24 for the device 1.

The received status signal 24 is indicative of a motion status of the object of interest 7, for example with respect to motion that is caused by the movement of the beating heart itself. The mode determining unit 3 may analyze the motion status of the object of interest 7. Based on that analysis, the mode determining unit 3 may determine an acquisition mode for an X-ray pulse for X-ray imaging apparatus 10. For the determination of the acquisition mode, the mode determining unit 3 determines at least one of the imaging parameters which may, for example, be selected from the group of the focal spot size, the pulse duration and the X-ray intensity, for the next X-ray pulse.

In a further exemplary embodiment, if the object of interest 7 is a living object, prior to the procedure a user may specify a region of interest, and this region is the topic of the time segmentation with respect to the signal of the status acquisition apparatus 5. Based on predetermined information as may be stored in a dedicated data base, the speed of an object in the selected region in relation to a signal of the status acquisition apparatus 5 is determined for example as a three-dimensional vector. The dependency of the vector to the signal of the status acquisition apparatus in combination with an actual beam angle setting during the procedure, is used to determine the optimal time segmentation of small and large focal spot use, respectively.

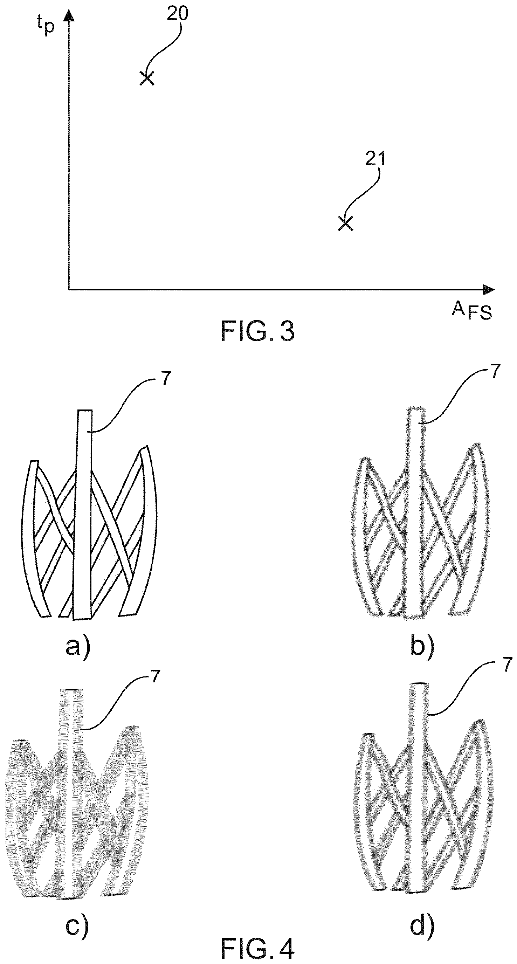

Fig. 3 shows a diagram wherein the vertical axis shows the pulse duration t_{P} and the horizontal axis shows the focal spot size A_{FS} for an exemplary embodiment in which the device 1 determines the focal spot size and the pulse duration. Two pairs 20, 21 of the pulse duration and focal spot size are marked in the diagram. The pair 20 shows a long pulse duration in combination with a small focal spot size. The pair 21 shows a short pulse duration and a large focal spot size.

The mode determining unit 3 may provide a small focal spot size in the range of 0.1 mm to 0.6 mm, preferably 0.3 mm to 0.5 mm for an X-ray pulse during a rest phase 23 of an object of interest 7. During a movement phase 22, the mode determining unit 3 may provide a large focal spot size in the range of 0.6 mm to 1.5 mm, preferably 0.7 mm to 1.2 mm as acquisition mode for an X-ray pulse.

The pulse durations may for example be in the range between 1 ms and 15 ms, dependent on the predetermined dose level to be set for the imaging procedure. For example, if a higher contrast-to-noise ratio is desirable, a longer pulse duration may be selected.

Furthermore, the mode determining unit 3 maybe configured to provide a substantially constant dose for all X-ray pulses during all motion statuses. Thus, the dose of X-ray radiation may be kept constant during an imaging sequence and does not vary between different X-ray pulses.

The mode determining unit 3 provides a mode signal which is indicative of the determined acquisition mode. Thus,the mode signal may comprise information about at least one of the group of the focal spot size, the pulse duration, and the X-ray intensity for the next X-ray pulse.

The mode signal may be transmitted or provided, for example by means of a transmitting unit 4, to the mode signal receiving unit 13 of the sequential X-ray imaging apparatus 10.

Alternatively, or in addition to an adaptation of the pulse duration, the dose of an X-ray pulse may be adapted by amending the X-ray tube voltage and/or the X-ray tube current. In an example, an adaptation of the tube voltage (kVp) may be considered, as this can be effectuated faster than an adaptation of the tube current (mA). Thus, an X-ray dose for different pulses may be kept constant, compensating a change in pulse duration with a corresponding change in kVp or mA.

Thus, in an example, in a rest phase of the object, a small focal spot size as described above may be combined with a relatively long pulse duration and/or a lower kVp, while in a motion phase a large focal spot size may be combined with a relatively short pulse duration and/or a higher kVp setting.

The tube voltage may be varied between a peak voltage of 20 kVp and 150 kVp, preferably adapted to standard protocols recommended for an interventional procedure.

The tube current may be varied between 10 mA and 2000 mA, preferably adapted to standard protocols recommended for an interventional procedure.

In an exemplary embodiment, the dose may then be adapted by amending the pulse duration and further amending the tube voltage and/or the tube current, wherein the focal spot size may be varied. If the tube voltage and the pulse duration are used for providing a constant dose for an X-ray pulse, then a dose reduction due to a lowered pulse duration is e.g. compensated by an e.g. non-linear dose raise due to a higher tube voltage. If the tube current and the pulse duration are used for providing a constant dose for an X-ray pulse, then the dose is kept constant by e.g. keeping a constant product of pulse duration and tube current.

In a further exemplary embodiment, the tube voltage, the tube current, and the pulse duration maybe varied to keep the dose constant, wherein the focal spot size may be varied. Then, a constant dose for an X-ray pulse is established by varying tube voltage, tube current and pulse duration, e.g. a dose reduction due to a lowered pulse duration is compensated by an, e.g. non-linear, dose raise due to a higher tube voltage and a higher tube current.

The controller 14 may adjust the tube current, and/or the tube voltage, and/or the pulse duration at the X-ray tube 12 to control the dose for an X-ray pulse. Furthermore, the controller 14 may adjust the focal spot size by controlling an electron beam focusing element in the X-ray tube.

Furthermore, a dose control definition for a small as well as a large focal spot size may be performed. In a first exemplary embodiment an independent dose regulation on the same detector dose may be performed. This embodiment provides a fast switching capability for the tube voltage and/or the tube current. In a further exemplary embodiment, if the tube current setting cannot be regulated adequately fast in the X-ray tube 12, the tube voltage is regulated, only. A long pulse duration with a low tube voltage and/or tube current is preferred for a small focal spot size, while a large focal spot size is preferred for short pulse duration with higher tube voltage and/or tube current setting.

Furthermore, a post processing of the acquired X-ray images with respect to the simultaneous usage of a small and a large focal spot size is performed. Furthermore, corrections which may potentially arise from small positioning tolerances of the focal spot may be corrected.

In case that different dose levels need to be used, a normalization of averaged intensity and a noise reduction of the X-ray images may be performed.

Fig. 4 shows four different imaging results of an object of interest 7 in two different motional phases. In this example, the object of interest 7 is a stent which is introduced into a pulsating vasculature. In Fig. 4 a) and b), the vasculature is in the rest phase 23, i.e. the stent being the object of interest 7 is also a rest phase 23. In Fig. 4 c) and d), the vasculature is in a movement phase 22, i.e. the stent is also moving.

Fig. 4 a) and 4 c) have been imaged with a small focal spot size and a long pulse duration, wherein Fig. 4 b) and 4 d) have been imaged with a large focal spot size and a short pulse duration.

When comparing Fig. 4 a) and b), Fig. 4 a) has a better quality than Fig. 4 b). This shows, that in a rest phase 23, an acquisition mode having a small focal spot size and a long pulse duration is suited best for a good image quality.

When comparing Fig. 4 c) and d), Fig. 4 d) has a better quality than Fig. 4 c). This shows, that in a movement phase 22, an acquisition mode having a large focal spot size and a short pulse duration is suited best for a good image quality.

Fig. 6 shows a schematic flow chart for the method 100 for determining a focal spot size and a pulse duration for an X-ray pulse for a sequential X-ray imaging apparatus.

In a first step d), a region of interest at the object of interest 7 may be specified 101 via a user interface. Thus, if the object of interest 7 is a living object, prior to the procedure a user may specify a region of interest, and this region is the topic of the time segmentation with respect to the signal of the status acquisition apparatus 5. Based on dedicated data bases, the speed of the selected region in relation on a signal of the status acquisition apparatus 5 is determined in a three-dimensional vector. This vector is brought into relation to the signal of the status acquisition apparatus. The dependency of the vector to the signal of the status acquisition apparatus in combination with an actual beam angle setting during the procedure, is used to determine the optimal time segmenting of small and large focal spot use, respectively.

In a further step e), a status acquisition apparatus may be adjusted 102 to the region of interest. The status acquisition apparatus 5 which may e.g. be an electrocardiograph apparatus comprises a sensor 51 which is connected to the chest of the patient 6 being arranged on the patient support device 8. In the exemplary embodiment being shown in Fig. 1, the status acquisition apparatus 5 monitors the heartbeat of the patient 6. The movement status of the heart may be determined from the monitoring of the heartbeat. The signal of the electrocardiograph apparatus comprises a movement phase 22, which indicates a movement of the heart during a heartbeat, and a rest phase 23 indicating a non-moving phase of the heart between two heart beats. Step e) may be performed by arranging a sensor 51 of a mode acquisition apparatus 5 to a chest of a patient 6, when the object of interest 7 is in the chest of the patient 6.

The status acquisition apparatus 5 may provide the signal of the electrocardiograph apparatus as a status signal 24 being shown in Fig. 5. The status signal 24 may be received by the device 1. According to Fig. 2, device 1 comprises a receiving unit 2, a mode determining unit 3, and a transmitting unit 4.

In the further step a), the receiving unit 2 may receive the status signal 24 for the device 1.

The received status signal 24 indicates a motion status of the object of interest 7.

Then, in a further step b), an acquisition mode based on the indicated motion status of the object of interest may be determined 104 with a mode determining unit.

The mode determining unit 3 may analyze the motion status of the object of interest 7. Based on that analysis, the mode determining unit 3 may determine an acquisition mode for an X-ray pulse for a sequential X-ray imaging apparatus 10. For the determination of the acquisition mode, the mode determining unit 3 determines at least one of the group of the focal spot size, a pulse duration, and the X-ray intensity for the next X-ray pulse. It is not necessary to adapt the frequency for the provisioning of the X-ray pulse is to a frequency of an oscillatory movement of the object of interest 7.

However, it is not excluded, that the frequency for the provisioning of the X-ray pulses is adapted to the frequency of an oscillatory movement of an object of interest 7. In a further method step c), the mode signal may be transmitted 105 to the mode signal receiving unit 13 of the sequential X-ray imaging apparatus 10, for example by means of a transmitting unit 4.

The method 100 may be performed with a system 30 for determining imaging parameters such as a focal spot size, and/or a pulse duration, and/or an X-ray intensity for an X-ray pulse for a sequential X-ray imaging apparatus, as has been described in the above with reference to Fig. 1.

In case that different dose levels need to be used for the X-ray images of a set of X-ray images, step f) maybe performed by normalizing an averaged intensity of 106 a set of X-ray images being acquired with a sequential X-ray imaging apparatus with different focal spot sizes and different doses for the respective X-ray pulses.

Furthermore, a noise reduction of the X-ray images may be applied 107 to the set of X-ray images.

In another exemplary embodiment of the present invention, a computer program or a computer program element is provided that is characterized by being adapted to execute the steps of the method according to one of the preceding embodiments, on an appropriate system. Thus, in this embodiment, the method is a computer-implemented method.

The computer program element might therefore be stored on a computer unit, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated, and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A device for determining imaging parameters for generating an X-ray pulse in an X-ray imaging apparatus, the device (1) comprising:
- a receiving unit (2) configured to receive a status signal (24) indicative of a motion status of an object of interest (7) ,and
- a mode determining unit (3) configured to determine an acquisition mode defining one or more imaging parameters for generating the X-ray pulse;
wherein the one or more imaging parameters are defined in dependence of the indicated motion status of the object of interest (7).

2. Device according to claim 1, wherein the one or more imaging parameters are selected from a group comprising a focal spot size, a pulse duration and an X-ray intensity for the X-ray pulse.

3. Device according to claim 2, wherein the mode determining unit (3) is configured to provide a smaller focal spot size and/or a longer pulse duration if the status signal (24) indicates a rest phase (23) as the motion status than if the status signal (24) indicates a movement phase (22) as motion status.

4. Device according to claim any preceding claim, wherein the mode determining unit (3) is configured to define the one or more imaging parameters so as to provide a constant X-ray pulse dose for X-ray pulses in different motion statuses.

5. Device according to claim 4, wherein the dose of an X-ray pulse is set by means of adapting a pulse duration in combination with adapting an X-ray tube voltage and/or an X-ray tube current.

6. An X-ray imaging system (30) comprising:
- an X-ray imaging apparatus (10);
- a status acquisition apparatus (5); and
- a device (1) according to one of the preceding claims;
wherein the X-ray imaging apparatus (10) comprises:
- an X-ray tube (12) and
- a controller (14);
wherein the status acquisition apparatus (5) is configured to determine a motion status of an object of interest (7) and to provide the status signal (24) indicative of the motion status to the device (1);
wherein the controller (14) is configured to control the X-ray tube (12) according to the one or more imaging parameters of the acquisition mode as determined by the device (1).

7. System according to claim 6, wherein the status acquisition apparatus (5) is an electro-cardiograph apparatus.

8. System according to claim 6 or 7, wherein the controller is configured to adjust a tube voltage of the X-ray tube (12) when controlling an X-ray intensity for an X-ray pulse.

9. System according to one of claims 5 to 7, wherein the status acquisition apparatus (5) is configured to provide the status signal (24) comprising a rest phase (23) segment and a movement phase (22) segment.

10. A method for determining imaging parameters for generating an X-ray pulse in an X-ray imaging apparatus, the method (100) comprising the following steps:
a) receiving (103) a status signal indicative of a motion status of an object of interest;
b) determining (104) an acquisition mode including defining one or more imaging parameters of the acquisition mode in dependence of the indicated motion status of the object of interest.

11. Method according to claim 10, wherein prior to step a) the method (100) further comprises the steps:
d) specifying (101) a region of interest at an object of interest via a user interface; and
e) adjusting (102) a status acquisition apparatus to the region of interest.

12. Method according to one of claims 10 or 11, wherein the method further comprises the steps:
f) normalizing (106) the signal in a sequence of X-ray images being acquired in a pulsed acquisition with an X-ray imaging apparatus, using different focal spot sizes and different doses for the respective X-ray pulses; and
g) applying (107) a noise reduction algorithm to the sequence of X-ray images.

13. A computer program element (41) comprising instructions for controlling a device according to one of the claims 1 to 5 or a system according to one of claim 6 to 9, wherein the instructions, when being executed by a processing unit of a computer, cause the computer to perform the method steps of one of the claims 10 to 12.

14. A computer readable medium (40) having stored the program element (41) of claim 13.
